# EUROPEAN PATENT SPECIFICATION

(11) **EP 0 555 412 B1**
(45) Date of publication and mention of the grant of the patent: **08.01.1997**
(21) Application number: 92902585.6
(22) Date of filing: 30.10.1991
(51) Int. Cl.: A61M 25/01

(54) **OVER-THE-WIRE CATHETER**
KATHETER MIT FÜHRUNGSDRAHT
CATHETER SUR FIL

(30) Priority: 31.10.1990 US 608110
(43) Date of publication of application: 18.08.1993
(73) Proprietor: BAXTER INTERNATIONAL INC. (a Delaware corporation), Deerfield Illinois 60015 (US); DEN HEIJER, Peter, NL-9751 AA Haren (NL); TEIRSTEIN, Paul S., La Jolla, CA 92037 (US)
(72) Inventor: DEN HEIJER, Peter, NL-9751 AA Haren (NL); TEIRSTEIN, Paul, S., La Jolla, CA 92037 (US); SIRIMANNE, D., Laksen, Tustin CA 92680 (US); CUTTER, Sandra, L., Mission Viejo, CA 92691 (US); KEMKEMIAN, Zohrab, Sarkis, West Covina, CA 91790 (US)
(74) Representative: MacGregor, Gordon
(86) International application number: US9108053
(87) International publication number: WO9207608

(56) References cited:
- EP-A- 0 165 727
- EP-A- 0 244 955
- US-A- 4 790 331

## Description

### BACKGROUND OF THE INVENTION

The invention generally relates to a catheter system for performing transluminal angioplasty procedures and the like and is more particularly directed to a steerable dilatation catheter having advantages not found in conventional "fixed wire" catheters or "over-the-wire" catheters.

A number of vascular conditions may be treated by percutaneous transluminal coronary angioplasty in which a balloon catheter is routed through the vascular system and positioned across a stenotic lesion. The balloon is then inflated with a fluid to compress the lesion against the artery wall in order to increase its effective luminal diameter.

Apparatus suitable for such procedures currently available can be classified as either a "fixed wire" catheter system or an "over-the-wire" catheter system. A typical "fixed wire" catheter system such as described in EP-A-0 165 727 or U.S. Re. 33,160, dated February 20, 1990, includes a guide wire with a dilatation, or working, catheter permanently fixed thereto. The distinctive advantage of this type of catheter is its relatively low profile, or small overall outer diameter, which enables its use in smaller arteries and in situations with more advanced stenosis in which an artery may be closed to such an extent that a larger diameter catheter, such as an over-the-wire catheter, may not be suitable.

The fixed wire catheter system is not without disadvantage. Because the dilatation catheter is fixed to the guide wire at a distal end thereof which includes a flexible steering portion or tip, the tip itself is necessarily fixed, as well as it's length, thus limiting the steerability of the fixed wire catheter. In addition, since the fixed wire dilatation catheter system must be rotated, both the wire and the balloon are is subject to distortion, particularly "balloon wrapping", due to the rotation thereof which may result in non-uniform inflation, limited ability or even inability for inflation and deflation thereof. EP-A-0 165 727 which is considered to represent the closest prior art with respect to the present invention discloses a guide wire rotation limiter for minimising "balloon wrapping".

In addition, with a fixed wire system the working catheter cannot be replaced over the guide wire which can be left in situ to establish an established path through the vascular system as hereinafter described.

An over-the-wire catheter system, such as described in U.S. Patent No. 4,540,404, utilizes a separate guide wire. The guide wire may first be inserted into a vascular system, and thereafter, a dilatation, or working, catheter may be inserted thereover, until a balloon member thereof is positioned across the stenotic lesion. Advantageously, because the guide wire is not fixed to the working catheter, the guide wire and working catheter may be inserted and withdrawn independent of each other. Thus, if necessary, the guide wire can be manipulated independently through a particularly curvaceous artery. In addition, once the guide wire is in place, the working catheter may be replaced as may be required to change it for a working catheter with a different or larger balloon during the angioplasty procedure. In view of these advantages, the over-the-wire system has been considered more "user friendly" than a fixed wire system.

Similarly, because the guide wire may be independently inserted and removed from a vascular system, if a change in a tip shape, size or type is deemed necessary to facilitate advancement or access, the guide wire may be removed, leaving the working catheter in place which subsequently provides an open channel for the replacement guide wire. Once the guide wire is positioned past the stenotic lesion, the working catheter can then be positioned to place the balloon portion across the stenotic lesion for inflation. After deflation and removal of the working catheter, the guide wire may be left in place, enabling reinsertion or exchange of the balloon catheter if necessary for a repeated angioplasty procedure. Alternatively, if the guide wire is removed leaving the working catheter, the lumen thereof, through which the guide wire had passed, is now available for monitoring or other uses.

The present invention is directed to a fixable wire catheter having a number of advantages, not available in prior art devices, as will hereinafter be described in greater detail.

A catheter system in accordance with the present invention is set out in Claim 1, and includes a working catheter adapted for sliding along a guide wire within a vascular system. The working catheter can be inserted and moved within the vascular system with the guide wire and further enabling the guide wire to be rotated within the working catheter during advancement of the guide wire for steering the guide wire therethrough. This arrangement allows simultaneous insertion of the guide wire and working catheter without rotation of the latter, which rotation could result in damage to or distortion of the working catheter, such as "balloon wrapping" as hereinbefore described.

A catheter system in accordance with an embodiment of the present invention may also include a guide wire sized for insertion into an animal vascular system and having a proximal and a distal end. The distal end includes a means for advancing the guide wire through the vascular system which more particularly may include a flexible, yet shapable, end portion.

The means provided for enabling rotation of the guide wire within the working catheter allows the orientation and re-orientation of the shapable flexible end portion to positions suitable for advancing the guide wire, with the working catheter coupled thereto, through the vascular system.

The means for removably coupling the working catheter to the guide wire may include a compressible member having an opening therethrough which provides means for enabling passage of the guide wire and fluid therethrough when the compressible member is not compressed and the guide wire has not been inserted.

An advantage of the present invention is the control of the length of the guide wire tip portion extending beyond the working catheter caused by the relative longitudinal placement of the distal end of the guide wire with respect to the distal end of the working catheter.

### DESCRIPTION OF THE DRAWINGS

A better understanding of the present invention may be had in connection with the following detailed description, taken in conjunction with the accompanying drawings, in which:
Figure 1 is a side view of the catheter system in accordance with the present invention generally showing a guide wire, a working catheter, a Y-connector, and a torquing device unit to couple the working catheter to the guide wire as hereinafter described in greater detail;
Figure 2 is a cross-sectional view of the torqu ing device in accordance with the present invention;
Figures 3a, b, c, and d are diagrammatic illustrations of relative positions of the distal end of the guide wire having a shapable portion thereon, and the distal end of the working catheter showing how the working catheter may enable the steering of the guide wire in accordance with the present invention;
Figure 4 is a side view of the catheter system shown in Figure 1 with the torquing device separated from the Y-connector, enabling independent longitudinal movement of the guide wire within the catheter; and
Figure 5 is a side view of the present invention including an alternative standard type Y connector and showing how manipulation of the guide wire within the working catheter is enabled when a torquing device is decoupled from the Y connector.

### DETAILED DESCRIPTION

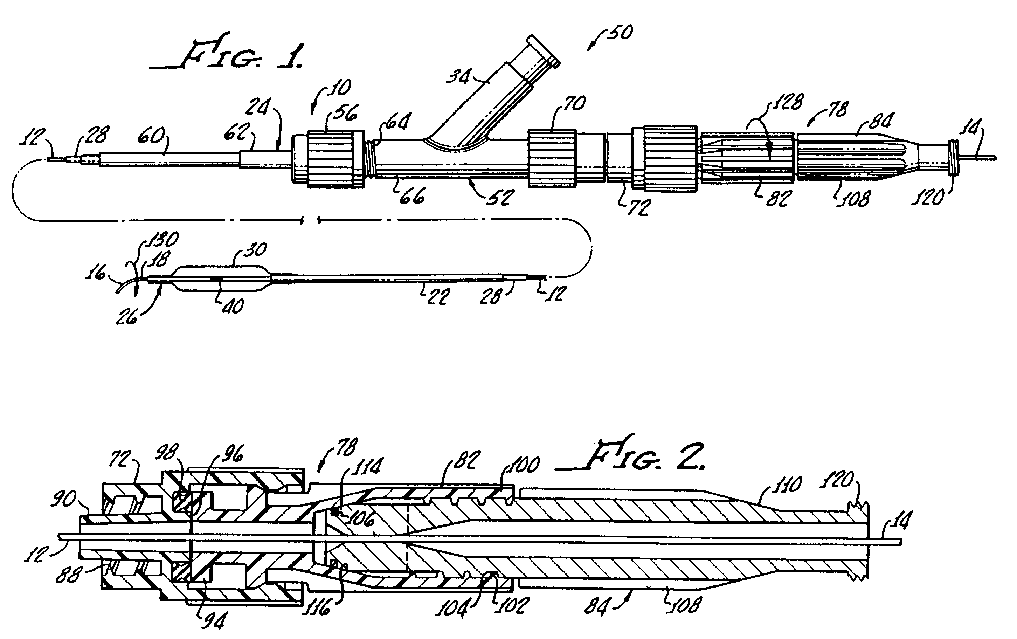

Turning now to Figure 1, there is shown a catheter system 10, including a guide wire 12, having a proximal end 14 and a distal end 16, the latter having a shapable flexible end portion 18 thereon, which may be of any suitable configuration, such as a fine, helically wound coiled wire or the like, and a working catheter 22 having a proximal end 24, a distal end 26, and an inside diameter 28.

Disposed at the distal end 26 of the working catheter 22 is an inflatable balloon 30 in a fluid communication with a port 34 via a working catheter lumen 36. The configuration of the working catheter 22, including balloon 30, as well as the guide wire 12, may be of any conventional design as is well known in the art. In addition, as is also well known in the art, a radiopaque marker 40 may be provided for use in determining the position of the distal end 26 of the working catheter 22 as it is advanced through a vascular system (not shown).

As shown in Figure 1, control apparatus 50 may be provided which includes a Y-connector 52, including the port 34 which provides means for inflating and deflating the balloon 30 by injecting or aspirating a fluid through the lumen 36 of the working catheter 22. The Y-connector 52, as well as a coupling 56 joining the working catheter 22 thereto, may be of conventional design. In addition, as shown in Figure 1, the outside diameter of the working catheter 22 may be increased in portions 60, 62 by the use of elastomeric coatings or metallic or polymeric sheathings for stiffening purposes to enhance the strength thereof

It can be appreciated that when the coupling 56 is attached, for example by means of threads 64, to the Y-connector body 66, the working catheter 22 is mounted in a non-rotatable relationship with the control apparatus 50. The guide wire 12, however, extending through the control apparatus 50, including couplings 70, adapter 72 and a torquing device 78, is rotatable within the working catheter 22 as will be explained hereinafter in greater detail.

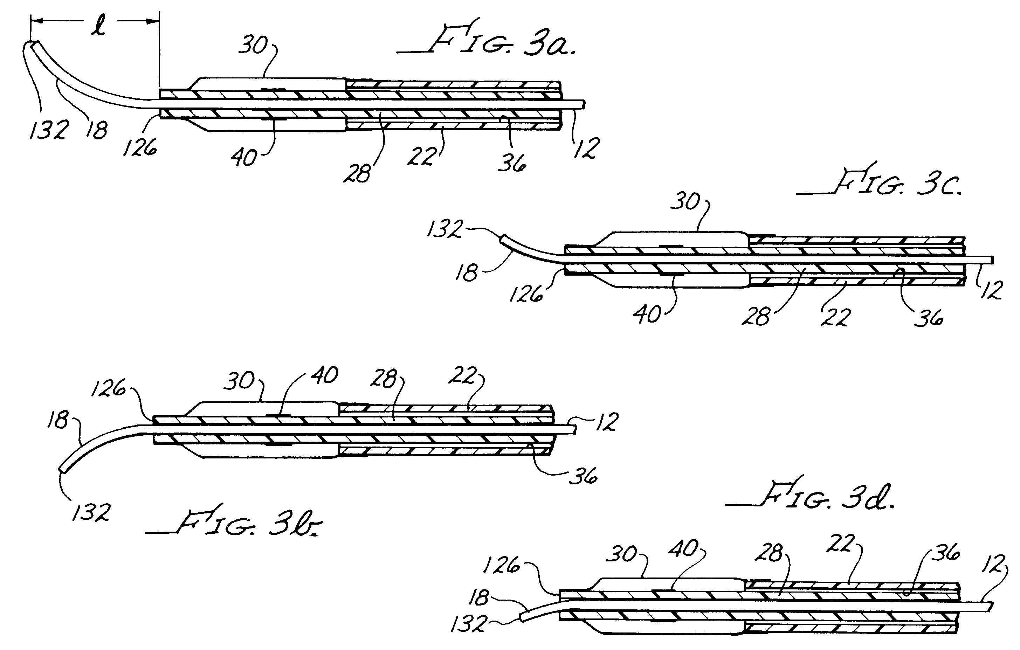

Turning now to Figure 2, there is shown in cross-section the torquing device 78, which is part of the control apparatus 50, generally showing the adapter 72, along with a sleeve 82, and a shaft 84.

The adapter includes internal threads 88 for engagement with the coupling 70 of the control apparatus and including a lumen 90 therethrough for communi cation with the lumen 28 of the working catheter 22.

Disposed within the adapter 72 for rotation therein is a sleeve head 94, having a face 96 pressed against a seal 98 for facilitating rotation.

The sleeve 82 includes a body 100 having threads 102 adapted for engagement with mating threads 104 on the shaft 84. When engaged, a seal 106 is pressed against the sleeve 94 to prevent a fluid which may be present in lumen 90 from passing therepast.

Ribs 108 on a body portion 110 facilitate the screwing of the shaft 84 into the sleeve 82 for compressing a compressible member, or tip 114, as it is forced against a tapered side diameter 116 of the sleeve 82, thus compressing the tip 114 onto the wire 12 and forming a seal for fluids within the lumen 90. Hence, it can be seen that the shaft 84 and the sleeve 82 and adapter 72 provide means for coupling the working catheter 22 to the guide wire 12 and also means for enabling rotation of the guide wire 12 within the working catheter 22. It is to be appreciated that since the working catheter 22 is affixed to the Y-coupling 52 along with the adapter 72, when the tip 114 is compressed against the guide wire 12, longitudinal sliding motion of the guide wire is prevented, but rotation within the working catheter 22 is made possible.

A luer thread 120 is provided on the body portion 110 and disposed in fluid communication with a central passage 122 of the shaft 84, which enables fluid to pass therethrough and into the lumen 90 of the adapter 72 and lumen 28 of the working catheter 22 for flushing or other purposes.

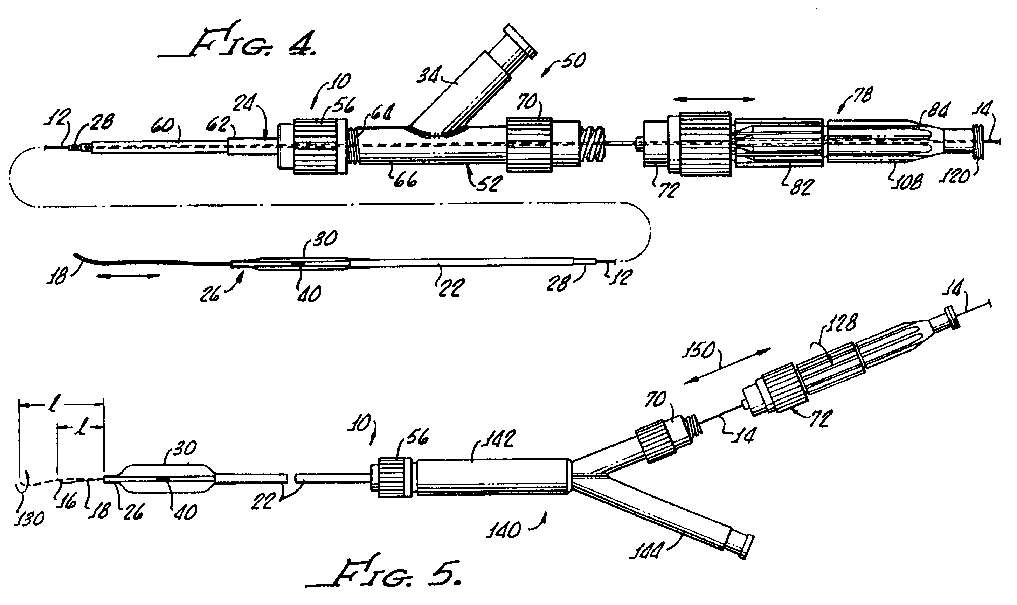

Referring now to Figure 1, working catheter 22, in operation, may be slid over the guide wire 12 until a selected length of the shapable flexible end portion 18 extends beyond a working catheter tip 126, see Figures 3a, b, c and d. At that point, the working catheter 22 is removably coupled to the guide wire 12 by rotation, or twisting, of the shaft 84 into the sleeve 82, thereby compressing the tip 114 onto the guide wire 12. The working catheter guide wire 12 is then inserted into a vascular system as in a conventional "fixed wire" system and thereafter, rotation of the sleeve 82, as shown by the arrow 128 (Figure 1), results in the rotation of the guide wire 12 within the working catheter 22 and the shapable flexible end portion 18 as shown by the arrow 130. This provides means for orienting and reorienting the shapable flexible end portion 18 to positions suitable for advancement of the guide wire 12 with the working catheter 22 coupled thereto, through the vascular system (not shown).

The shapable flexible end portion 18 includes a length thereof "l", which is determined by the relative longitudinal placement of a distal end tip 132 with respect to the distal tip 126 of the working catheter 22 as shown in Figure 3a. Figure 3b shows the same length, l, with the guide wire 12 end portion 18 oriented 180° from the orientation shown in Figure 3a. Such orientations facilitate the advancement and manipulation of the guide wire 12 and working catheter 22 through the vascular system.

As shown in Figures 3c and 3d, the working catheter 22 may be clamped to the guide wire 12 at a position at which the longitudinal placement of the distal tip 132 of the guide wire 12 with respect to the distal tip 126 of the working catheter 22 is substantially shorter, as may be necessary to facilitate advancement of the guide wire 12, with the working catheter 22 attached thereto, through various arcuate portions of the vascular system.

Turning to Figures 4 and 5, there is shown the torquer 78 decoupled with the Y-connector 52 and an alternative Y-connector 140 having a body 142 and port 144 for fluid access to the collector lumen 36 for inflating and deflating the balloon 30.

The torquing device 78 is shown decoupled to the Y-connectors 52, 140 in Figures 4 and 5. In this configuration the guide wire 14 may be moved in a sliding fashion within the working catheter 22 as indicated by the arrow 150, which results in adjusting the length, l, of guide wire 12 projecting beyond the working catheter distal tip 126. In this manner the guide wire 14 may be advanced within a vascular system ahead of the working catheter 22. Alternatively, when the torquing device 78 is coupled to either of the Y-members 52, 140, the working catheter 22 may be advanced with or, manipulated with, the guide wire 12.

A procedure for the insertion of a working catheter 22 through a vascular system includes the steps of inserting the guide wire 12, having the shapable end portion 18 into a vascular system (not shown). The shapable flexible end portion is used to direct, or steer, the guide wire 12 through the arcuate and bifurcated portions of the vascular system. Thereafter, the working catheter 22 is inserted over the guide wire 12 and coupled to the guide wire 12 with a selectable length, l, of the shapable flexible end portion 18 protruding from the working catheter 22.

Alternatively, the working catheter 22 may be disposed over the guide wire 12 and coupled thereto as hereinabove described before simultaneous insertion of the guide wire 12 and working catheter into the vascular system.

Thereafter, the guide wire 12 may be rotated within the working catheter 22 in order to orient the shapable flexible end portion to a position suitable for continued advancement of the guide wire 12 with the working catheter coupled thereto within the vascular system (see Figures 3a, b, c and d). With the proper orientation, the procedure then includes advancing the guide wire 12 and working catheter 22 in the vascular system.

## Claims

1. A catheter system comprising:
a guide wire (12) having a proximal end (14) and a distal end (16), the guide wire being received in a working catheter (22);
a torquing device (78) coupled to the guide wire for rotating the guide wire within the working catheter; and
coupling means (114) in a first position thereof coupling said guide wire with the working catheter to prevent the catheter from sliding axially over the guide wire;
characterised in that the coupling means (114) in the first position thereof does not limit rotatability of the guide wire (12) within the working catheter (22), and is movable to a second position thereof in which the coupling of the guide wire (12) with the working catheter (22) is removed so that the working catheter (22) is slidable axially over the guide wire (12), the torquing device (78) being removably coupled to the guide wire (12).

2. The catheter system according to Claim 1 wherein said torquing device (78) and coupling means (114) are disposed near the guide wire proximal end (14).

3. A catheter system according to Claim 1 or 2, wherein the distal end (16) of the guide wire (12) includes steering means (18) for enabling steering of said guide wire through a vascular system by rotation of the guide wire (12) therein.

4. The catheter system according to any preceding claim wherein said coupling means (114) includes a compressible member (114) having means, defining an opening, for enabling passage of said guide wire and fluid therethrough when said compressible member is not compressed onto the guide wire (12).

5. The catheter system according to Claim 3, or Claim 4 when dependent from Claim 3, wherein said steering means (18) includes a shapable flexible end portion (18) disposed at the distal end of said guide wire (12).

6. The catheter system of any preceding claim, wherein the working catheter (22) comprises balloon means (30), disposed at the distal end (26) thereof, for compressing atherosclerosis when the catheter is within an animal vascular system, a balloon inflation means (34,36) being disposed near the proximal end (14,24) of the guide wire and the working catheter for inflating and deflating the balloon means by injecting or aspirating a fluid through the working catheter, the catheter system including means for injecting or aspirating fluid between said guide wire (12) and said working catheter (22).

## Patentansprüche

1. Kathetersystem, das folgendes aufweist:
einen Führungsdraht (12), der ein proximales Ende (14) und ein distales Ende (16) hat und in einem Arbeitskatheter (22) aufgenommen ist;
eine Dreheinrichtung (78), die mit dem Führungsdraht gekoppelt ist, um den Führungsdraht im Inneren des Arbeitskatheters zu drehen; und
eine Kopplungseinrichtung (114), die in einer ersten Position davon den Führungsdraht mit dem Arbeitskatheter koppelt, um den Katheter an einem axialen Gleiten über den Führungsdraht zu hindern;
dadurch gekennzeichnet, daß die Kopplungseinrichtung (114) in ihrer ersten Position die Drehbarkeit des Führungsdrahts (12) im Inneren des Arbeitskatheters (22) nicht begrenzt und in eine zweite Position davon bewegbar ist, in der die Kopplung zwischen dem Führungsdraht (12) und dem Arbeitskatheter (22) aufgehoben ist, so daß der Arbeitskatheter (22) axial über den Führungsdraht (12) gleitbar ist, wobei die Dreheinrichtung (78) abnehmbar mit dem Führungsdraht (12) gekoppelt ist.

2. Kathetersystem nach Anspruch 1, wobei die Dreheinrichtung (78) und die Kopplungseinrichtung (114) nahe dem proximalen Ende (14) des Führungsdrahts angeordnet sind.

3. Kathetersystem nach Anspruch 1 oder 2, wobei das distale Ende (16) des Führungsdrahts (12) eine Lenkeinrichtung (18) aufweist, um ein Lenken des Führungsdrahts durch ein Gefäßsystem mittels Rotation des Führungsdrahts (12) darin zu ermöglichen.

4. Kathetersystem nach einem der vorhergehenden Ansprüche, wobei die Kopplungseinrichtung (114) ein kompressibles Element (114) aufweist, das eine Öffnung definierende Mittel hat, um den Durchtritt des Führungsdrahts und von Fluid zuzulassen, wenn das kompressible Element nicht an den Führungsdraht (12) gepreßt wird.

5. Kathetersystem nach Anspruch 3, oder nach Anspruch 4 in Abhängigkeit von Anspruch 3, wobei die Lenkeinrichtung (18) einen formbaren biegsamen Endbereich (18) aufweist, der an dem distalen Ende des Führungsdrahts (12) angeordnet ist.

6. Kathetersystem nach einem der vorhergehenden Ansprüche, wobei der Arbeitskatheter (22) folgendes aufweist: eine Balloneinrichtung (30), die an seinem distalen Ende (26) angeordnet ist, um Atherosklerose zusammenzupressen, wenn sich der Katheter in einem animalen Gefäßsystem befindet, eine Ballonfülleinrichtung (34, 36), die nahe dem proximalen Ende (14, 24) des Führungsdrahts und des Arbeitskatheters angeordnet ist, um die Ballopeinrichtung durch Einpressen oder Absaugen eines Fluids durch den Arbeitskatheter zu füllen und zu entleeren, wobei das Kathetersystem Mittel aufweist, um Fluid zwischen dem Führungsdraht (12) und dem Arbeitskatheter (22) einzupressen oder abzusaugen.

## Revendications

1. Système de cathéter comprenant :
un fil de guidage (12) ayant une extrémité proximale (14) et une extrémité distale (16), le fil de guidage étant reçu dans un cathéter de travail (22) ;
un dispositif d'application de couple (78), couplé au fil de guidage pour faire tourner le fil de guidage à l'intérieur du cathéter de travail ; et
un dispositif de couplage (114) qui accouple, dans sa première position, ledit fil de guidage au cathéter de travail pour empêcher le cathéter de coulisser axialement sur le fil de guidage ;
caractérisé en ce que le dispositif de couplage (114), dans la première position,ne limite pas la possibilité de rotation du fil de guidage (12) à l'intérieur du cathéter de travail (22), et il est déplacable à une deuxième position dans laquelle le couplage du fil de guidage (12) avec le cathéter de travail (22) est supprimé de sorte que le cathéter de travail (22) peut coulisser axialement sur le fil de guidage (12), le dispositif d'application de couple (78) étant couplé de façon séparable au fil de guidage (12).

2. Système de cathéter suivant la revendication 1, dans lequel ledit dispositif d'application de couple (78) et le dispositif de couplage (114) sont prévus près de l'extrémité proximale (14) du fil de guidage.

3. Système de cathéter suivant la revendication 1 ou 2, dans lequel l'extrémité distale (16) du fil de guidage (12) comprend des moyens d'orientation (18) pour permettre l'orientation dudit fil de guidage dans un système vasculaire par rotation du fil de guidage (12) dans celui-ci.

4. Système de cathéter suivant une quelconque des revendications précédentes, dans lequel ledit dispositif de couplage (114) comprend un élément compressible (114) ayant des moyens de définition d'un orifice pour permettre le passage dudit fil de guidage et du fluide lorsque ledit élément compressible n'est pas comprimé sur le fil de guidage (12).

5. Système de cathéter suivant la revendication 3, ou la revendication 4 lorsqu'elle dépend de la revendication 3, dans lequel lesdits moyens d'orientation (18) comprennent une partie d'extrémité flexible configurable (18) prévue à l'extrémité distale dudit fil de guidage (12).

6. Système de cathéter suivant une quelconque des revendications précédentes, dans lequel le cathéter de travail (22) comprend un ballonnet (30) disposé à son extrémité distale (26) pour comprimer une athérosclérose lorsque le cathéter est placé dans un système vasculaire animal, un dispositif de gonflage de ballonnet (34,36) étant prévu près de l'extrémité proximale (14,24) du fil de guidage et du cathéter de travail pour gonfler et dégonfler le ballonnet par injection ou aspiration d'un fluide par l'intermédiaire du cathéter de travail, le système de cathéter comportant des moyens d'injection ou d'aspiration du fluide entre ledit fil de guidage (12) et ledit cathéter de travail (22).
